# EUROPEAN PATENT APPLICATION

(11) **EP 1 253 143 A1**
(43) Date of publication of application: **30.10.2002**
(21) Application number: 01902738.2
(22) Date of filing: 02.02.2001
(51) Int. Cl.: C07D 271/10

(54) **1,3,4-OXADIAZOLE DERIVATIVES AND PROCESS FOR PRODUCING THE SAME**

(30) Priority: 03.02.2000 JP 2000026718
(71) Applicant: ONO PHARMACEUTICAL CO., LTD., Osaka-shi, Osaka 541-8526 (JP)
(72) Inventor: SUGIURA, Tsuneyuki, c/o Minase Research Institute, Mishima-gun, Osaka 618-85 (JP); MIYAZAKI, Tohru, c/o Minase Research Institute, Mishima-gun, Osaka 618-8585 (JP); HORIUCHI, Toshihide,Minase Research Institute, Mishima-gun, Osaka 618-8585 (JP)
(74) Representative: Henkel, Feiler, Hänzel
(86) International application number: JP0100742
(87) International publication number: WO01057005

(57) **Abstract**

A process for the preparation of compounds represented by formula (I) and (III): 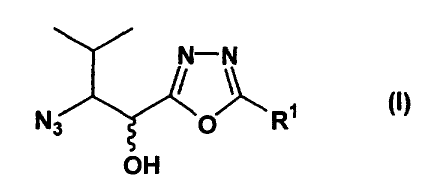 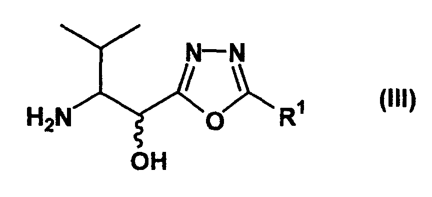 (wherein symbols in the formula are described in the description) and the novel 1,3,4-oxadiazole derivative compound represented by formula (I). According to the present invention, preparation of intermediates (compounds of formula (III)) of 1,3,4-oxadiazole derivatives useful as pharmaceuticals (elastase inhibitors) can be prepared efficiently via novel synthesis intermediates (compounds of formula (I)).

## Description

### TECHNICAL FIELD

The present invention relates to novel 1,3,4-oxadiazole derivatives and a process for the preparation thereof.

More particularly, the present invention relates to,
(1) a process for the preparation of 1,3,4-oxadiazole derivatives represented by formula (I) and (III): 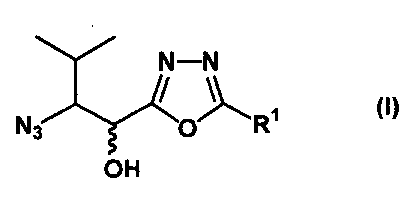 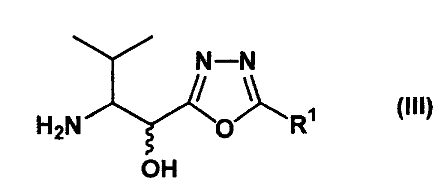 (wherein all symbols have the same meanings as described below.)
   which are synthesis intermediates of 1,3,4-oxadiazole derivatives useful as pharmaceuticals, and
(2) novel 1,3,4-oxadiazole derivatives represented by formula (I) obtained by the above preparation process.

### BACKGROUND OF THE INVENTION

As 1,3,4-oxadiazole derivatives useful as pharmaceuticals, for example,
(1) Published Japanese Translation of PCT International Publication for Patent Application No. 10-511933 discloses that compounds represented by formula (Z-a) to (Z-f): 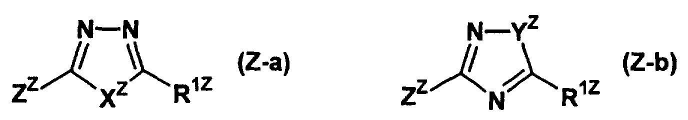 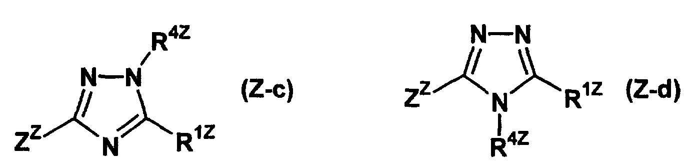 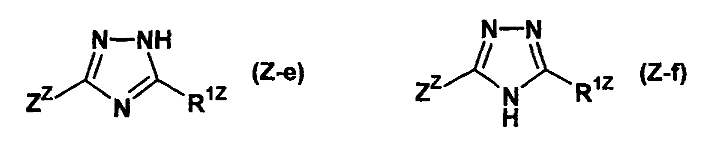 are useful as serine protease (particularly, elastase) inhibitors. However, the Preparation Process or Examples of the specification describe only on 1,2,4-oxadiazole but not on 1,3,4-oxadiazole.
(2) WO 98/24806 discloses that compounds represented by formula (W-a) to (W-c): 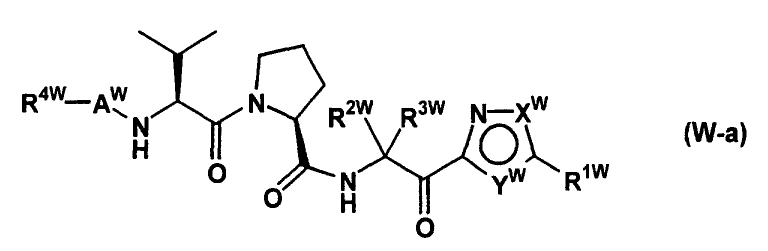 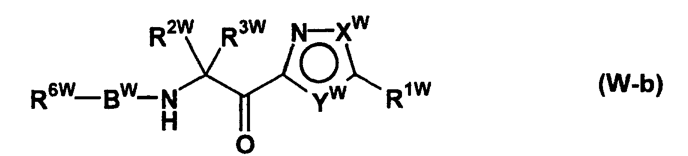 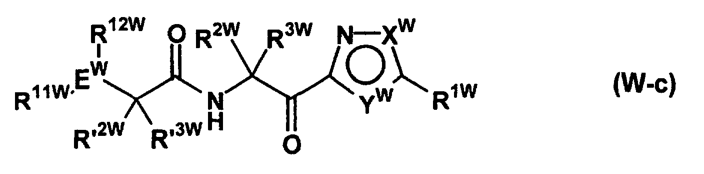 are useful as serine protease (particularly, elastase) inhibitors. In the specification, various compounds are synthesized via a compound represented by formula (W-1): 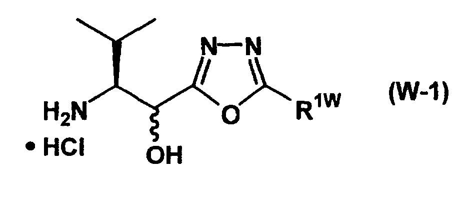 (in the formula, R^{1W} represents various substituting groups), and the compound is markedly important as an intermediate of pharmaceuticals.

Regarding the preparation process of the compound represented by formula (W-1), there is a description that it is prepared by the following reaction scheme 1 or 2.

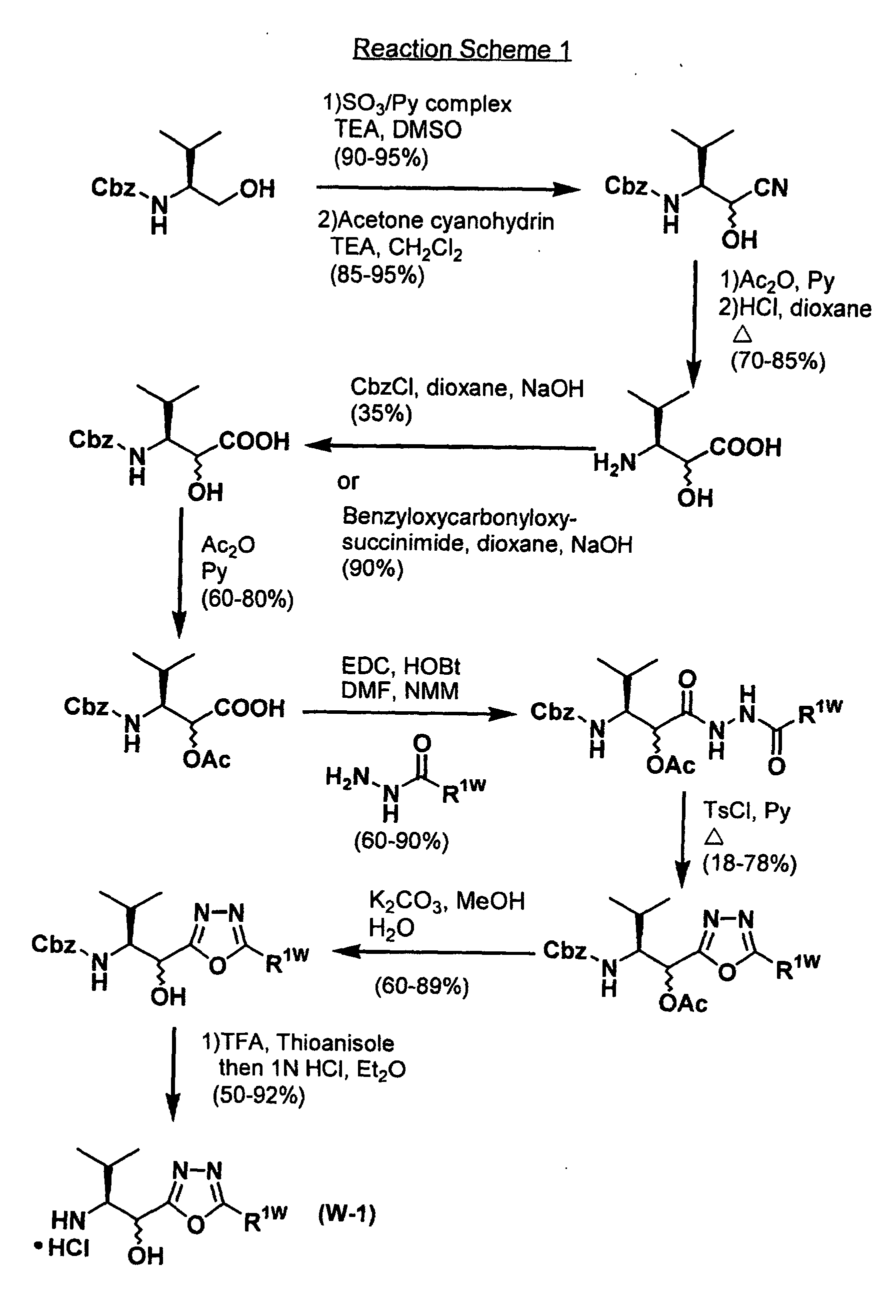

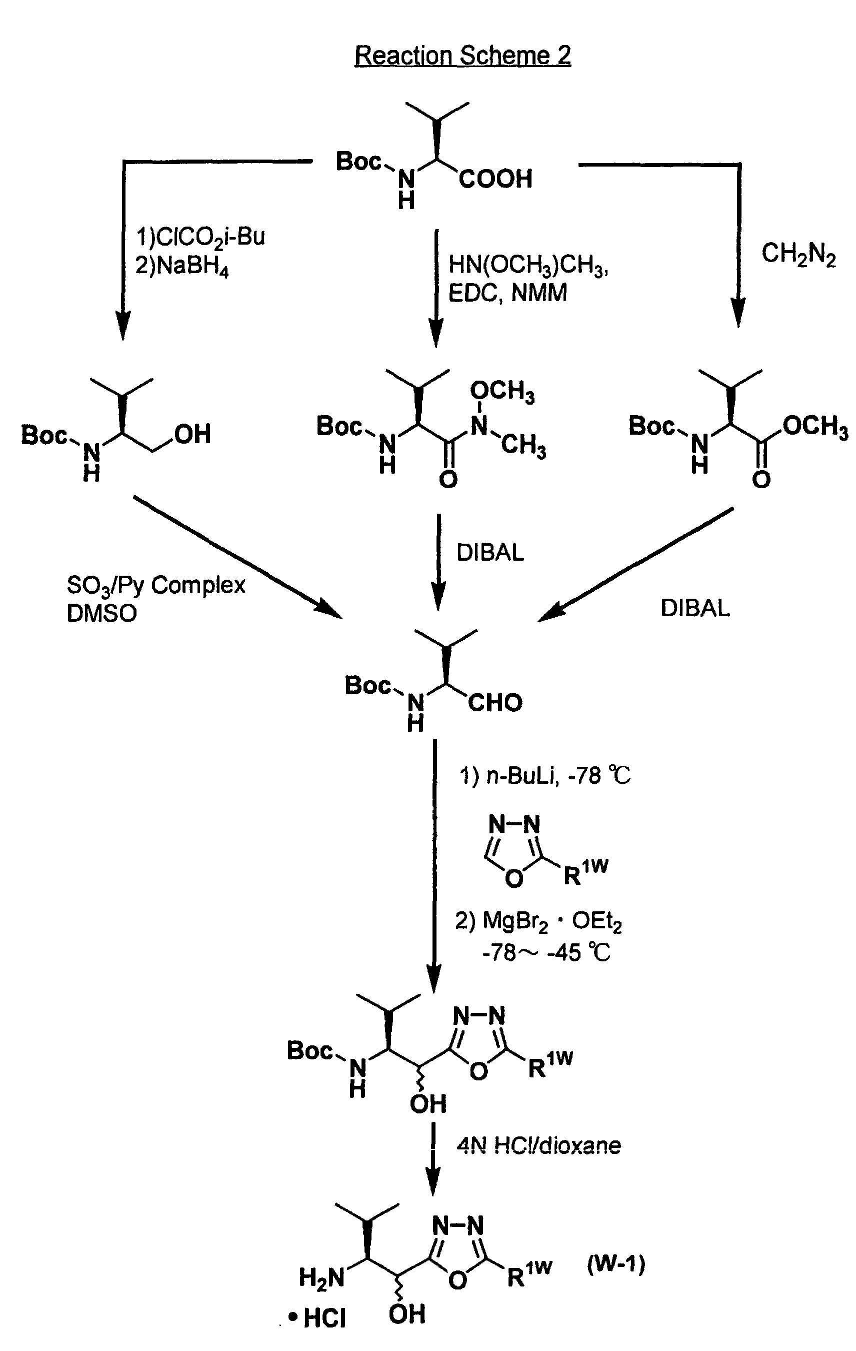

In the above reaction schemes,
R^{1W} represents various substituents,
Cbz represents a benzyloxycarbonyl group,
TEA represents triethylamine,
Ac represents an acetyl group,
Py represents pyridine,
EDC represents 1-ethyl-3-[3-(dimethylamino)propyl]carbodiimide,
HOBt represents 1-hydroxybenzotriazole,
NMM represents N-methylmorpholine,
TsCI represents p-toluenesulfonyl chloride,
Boc represents a t-butoxycarbonyl group, and
DIBAL represents diisobutylaluminum hydride.

In the conventional process shown by the reaction scheme 1, the compound represented by formula (W-1) is prepared from a material via a considerably large number of steps (10 steps), so that it was not sufficiently satisfactory as an industrial large scale synthesis process. Also, in the conventional processes shown by the reaction scheme 2, reactions at a low temperature (-78 to -45°C) are present so that they were not always satisfactory processes as industrial large scale synthesis processes. Accordingly, a process for the preparation of 1,3,4-oxadiazole derivatives which can be synthesized in an industrially large scale is required.

### DISCLOSURE OF THE INVENTION

Taking these problems involved in the background art into consideration, the present inventors have conducted intensive studies and found as a result of the efforts that a process shown by the following reaction scheme 3.

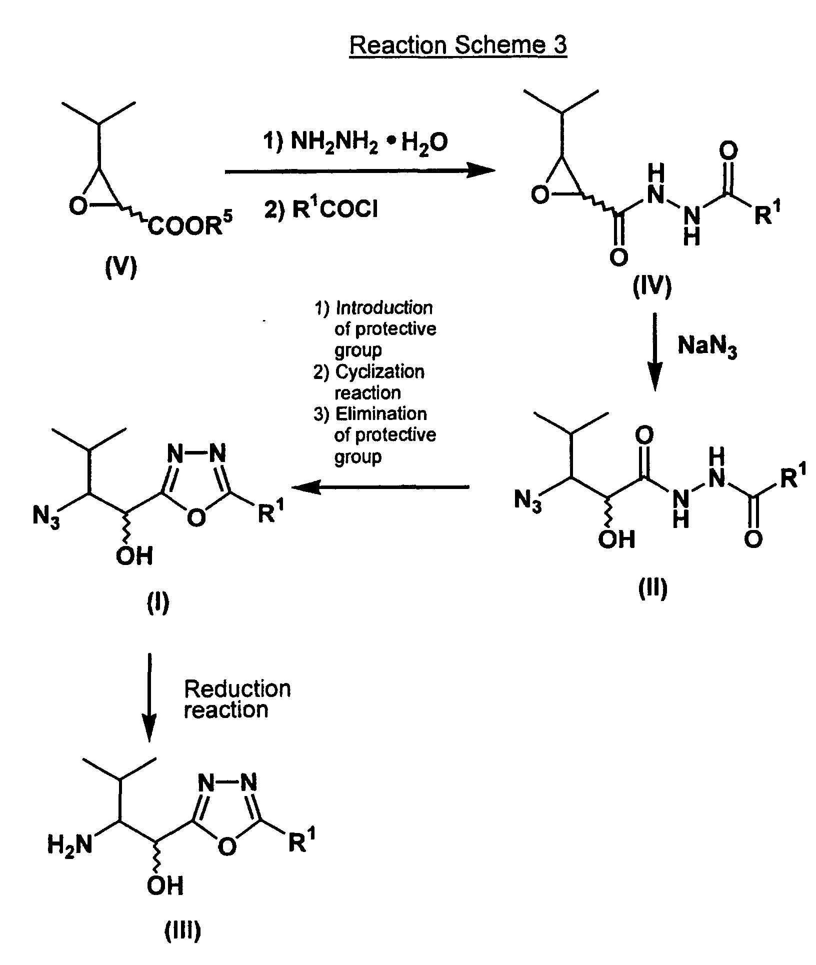

In the reaction scheme 3,
R¹ represents phenyl group or 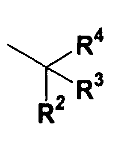 (wherein R², R³ and R⁴ each independently represents (1) a hydrogen atom, (2) a C1-8 alkyl group, (3) a C3-7 cycloalkyl group, (4) a phenyl group, (5) a phenyl group substituted with 1 to 3 substituents selected from a C1-8 alkyl group, a C1-8 alkoxy group, a halogen atom, a trifluoromethyl group and a trifluoromethoxy group or (6) a 3,4-methylenedioxyphenyl group, or (7) R² and R³ are taken together to represent a C2-6 alkylene group), and
R⁵ represents a methyl group or an ethyl group.

According to the process shown in the above reaction scheme 3, the compound represented by formula (III) which is important as an intermediate of pharmaceuticals can be prepared with a high yield by smaller steps without requiring a low temperature reaction.

Also, in the reaction scheme 3, the compound represented by formula (I), which is an important intermediate of known serine protease (particularly elastase) inhibitors, and the compounds represented by formula (IV) and formula (II) as synthesis intermediates thereof are novel compounds.

Accordingly, the present invention relates to
1) a process for the preparation of a 1,3,4-oxadiazole derivative represented by formula (I): 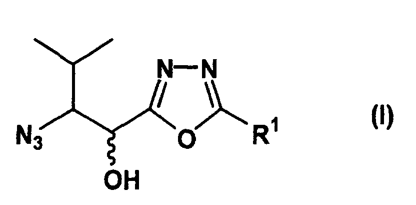 (wherein R¹ has the same meaning as described below), which comprises subjecting a compound represented by formula (II): 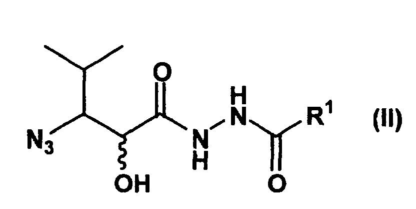 (wherein R¹ represents a phenyl group or 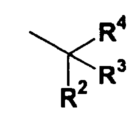 (wherein R², R³ and R⁴ each independently represents (1) a hydrogen atom, (2) a C1-8 alkyl group, (3) a C3-7 cycloalkyl group, (4) a phenyl group, (5) a phenyl group substituted with 1 to 3 substituents selected from a C1-8 alkyl group, a C1-8 alkoxy group, a halogen atom, a trifluoromethyl group and a trifluoromethoxy group or (6) a 3,4-methylenedioxyphenyl group, or (7) R² and R³ are taken together to represent a C2-6 alkylene group)) to an introducing reaction of a protecting group of a hydroxyl group, a cyclization reaction and a deprotection reaction of the protecting group of a hydroxyl group,
2) a process for the preparation of a 1,3,4-oxadiazole derivative represented by formula (III): 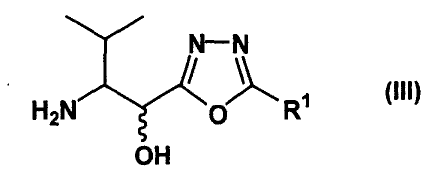 (wherein R¹ has the same meaning as described above) using the 1,3,4-oxadiazole derivative of formula (I) obtained by the above process, and
3) a novel 1,3,4-oxadiazole derivative represented by formula (I): 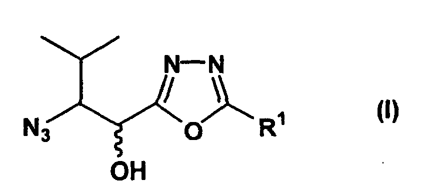 (wherein R¹ has the same meaning as described above).

### DETAILED DESCRIPTION OF THE INVENTION

In formula (I), the C1-8 alkyl groups represented by R², R³ and R⁴ include methyl, ethyl, propyl, butyl, pentyl, hexyl, heptyl and octyl groups and isomer groups thereof.

In formula (I), the C1-8 alkoxy groups represented by R², R³ and R⁴ include methoxy, ethoxy, propoxy, butoxy, pentyloxy, hexyloxy, heptyloxy and octyloxy groups and isomer groups thereof.

In formula (I), the C3-7 cycloalkyl groups represented by R², R³ and R⁴ include cyclopropyl, cyclobutyl, cyclopentyl, cyclohexyl and cycloheptyl groups and isomer groups thereof.

In formula (I), the C2-6 alkylene groups R² and R³ together represent include ethylene, trimethylene, tetramethylene, pentamethylene and hexamethylene groups and isomer groups thereof.

In formula (I), the halogen atoms represented by R², R³ and R⁴ include a fluorine atom, a chlorine atom, a bromine atom and an iodine atom.

According to the present invention, unless otherwise indicated and as is apparent for those skilled in the art, each of symbols 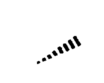 indicates that it is bound to the opposite side of the sheet (namely α- configuration), 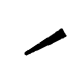 indicates that it is bound to the front side of sheet (namely β- configuration), 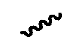 indicates that it is α-, β- or a mixture thereof, or E-isoform, Z-isoform or a mixture thereof, and 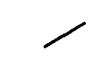 indicates that it is a mixture of α-configuration and β-configuration.

Unless otherwise indicated, all of these isomers are included in the present invention. For example, straight chains and branched chains are included in the alkyl groups, the alkoxy groups and the alkylene groups. In addition, all of isomers based on a double bond, a ring and a condensed ring (E-, Z-, cis- and trans-isomer), isomers due to the presence of an asymmetric carbon (R- and S-configuration, α- and β-configuration, enantiomers and diastereomers), optically active isomer having optical activity (D-, L-, d- and I-isomer), polar compounds obtained by chromatographic separation (more polar compounds and less polar compounds), equilibrium compounds, mixtures thereof at optional ratio and racemic mixtures are included in the present invention.

### Preparation process of the compound of the present invention

The compound of the present invention represented by formula (I) can be prepared by the following process or the process described in Examples.

That is, the compound of the present invention represented by formula (I): 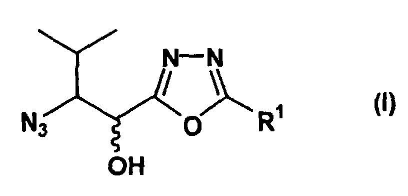 (wherein R¹ has the same meaning as described above) can be prepared by subjecting the compound represented by formula (II): 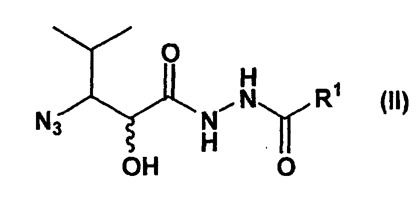 (wherein R¹ has the same meaning as descried above) to an introducing reaction of a protecting group of a hydroxyl group, a cyclization reaction and a deprotection reaction of the protecting group of a hydroxyl group.

The reaction for introducing a protecting group of a hydroxyl group is well known. For example, in the case of a silyl protecting group, the reaction is carried out in an organic solvent (acetonitrile, tetrahydrofuran, diethyl ether, dimethylformamide, or the like) at a temperature of from 0 to 50°C in the presence of a silyl compound (trimethylsilyl chloride, t-butyldimethylsilyl chloride, t-butyldiphenylsilyl chloride, or the like) and a base (pyridine, triethylamine, imidazole, or the like).

The cyclization reaction is well known (cf., *Tetrahedron Letters,* 267 (1976)), and the reaction is carried out in an organic solvent (tetrahydrofuran, diethyl ether, or the like) at a temperature of from 0 to 50°C in the presence of thionyl chloride and further continuing the reaction in an organic solvent (benzene, toluene, or the like) at a temperature of from 70 to 150°C.

The deprotection reaction of the protecting group of a hydroxyl group is well known. For example, in the case of a silyl protecting group, the reaction is carried out in a water-miscible organic solvent (tetrahydrofuran, acetonitrile, methanol or the like) at a temperature of from 0 to 40°C using a fluorine compound (tetrabutylammonium fluoride, potassium fluoride, or the like).

The group of a hydroxyl group is not particularly limited to the silyl group, and any group capable of being easily and selectively eliminated can be used. For example, a methoxymethyl group, a 2-tetrahydropyranyl group, an acetyl group, a benzyl group and those which are described by T.W. Greene, *Protective Groups in Organic Synthesis,* Wiley, New York, 1991, can be used.

The compound of the present invention represented by formula (III) which is important as an intermediate of pharmaceuticals, or optionally salts thereof can be prepared by the following process or the process described in Examples using the compound represented by formula (I).

That is, the compound represented by formula (III): 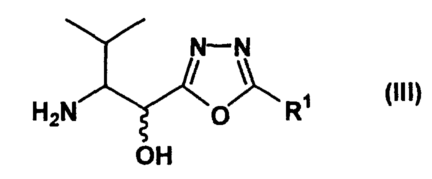 (wherein R¹ has the same meaning as described above) can be prepared by subjecting the compound of the present invention represented by formula (I) to a reduction reaction.

The reduction reaction is well known and can be carried out by a reduction reaction using a hydrolysis reaction.

The hydrolysis reaction is well known, and a deprotection reaction by the hydrolysis is carried out, e.g., at a temperature of from 0 to 200°C in an inert solvent [an ether (e.g., tetrahydrofuran, dioxane, dimethoxyethane, diethyl ether, *etc.*), an alcohol (e.g., methanol, ethanol, *etc.*), a benzene (e.g., benzene, toluene, *etc*), an amide (e.g., dimethylformamide, *etc.*), water, ethyl acetate, acetic acid, a mixed solvent of two or more of them, or the like] in the presence of a hydrogenation catalyst (e.g., palladium-carbon, palladium black, palladium, palladium hydroxide, platinum-carbon, platinum dioxide, nickel, raney nickel, ruthenium chloride, *etc.*), in the presence or absence of an inorganic acid (e.g., hydrochloric acid, sulfuric acid, hypochlorous acid, boric acid, tetrafluoroboric acid, *etc.*) or of an organic acid (e.g., acetic acid, p-toluenesulfonic acid, oxalic acid, trifluoroacetic acid, formic acid, *etc.*) under a hydrogen at atmospheric pressure or pressurization, or in the presence of ammonium formate. When an acid is used, salt thereof may be used.

The compound represented by formula (II) can be prepared using formula (V) in accordance with the process of the following reaction scheme 3.

Also, the compound represented by formula (V) is a known compound or can be prepared by a known process (cf. *Synth. Comm.*, 14, 121 (1984))

A product of each reaction may be subjected to the subsequent reaction by carrying out isolation, washing, drying and purification at each step or without carrying out these operations, or subjected to the subsequent step by suspending it after appropriate operations. The reaction product by each reaction can be purified by conventional techniques such as distillation at atmospheric or reduced pressure, high performance liquid chromatography, thin layer chromatography or column chromatography using silica gel or magnesium silicate, washing and recrystallization.

The compound represented by formula (III) can be converted into acid addition products and hydrates thereof by known methods.

Examples of the salt as used herein include salts of alkali metals (potassium, sodium, etc.), salts of alkaline earth metals (calcium, magnesium, etc.), ammonium salts, salts of pharmacologically acceptably organic amines (tetramethylammonium, triethylamine, methylamine, dimethylamine, cyclopentylamine, benzylamine, phenetylamine, piperidine, monoethanolamine, diethanolamine, tris(hydroxymethyl)aminomethane, lysine, arginine and N-methyl-D-glucamine, *etc.*) and acid addition salts (inorganic acid salts such as hydrochloride, hydrobromide, sulfate, phosphate and nitrate, or organic acid salts such as acetate, trifluoroacetate, lactate, tartarate, oxalate, fumarate, maleate, citrate, benzoate, methanesulfonate, ethanesulfonate, benzenesulfonate, toluenesulfonate, isethionate, glucuronate, gluconate, *etc.*). These salts can be prepared by known methods.

In addition, the compounds described herein or salts thereof can also be converted into hydrates by known methods.

### BEST MODE FOR CARRYING OUT THE INVENTION

The present invention is described below in detail based on reference examples and examples, but the present invention is not limited thereto.

In the chromatography separation and TLC, the solvents shown in parentheses show eluting or developing solvents, and their ratio is volume ratio. The solvent shown in parentheses in NMR is a solvent used in the measurement.

### Reference Example 1

### N-(3-Isopropyl)glycidyl-N'-(2,2-dimethylpropionyl)hydrazine:

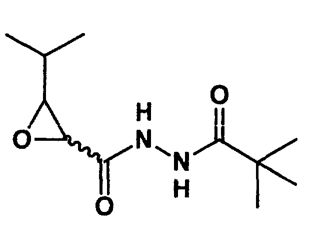

Hydrazine hydrate (27.8 g) was gradually added dropwise to methyl 3-isopropylglicidate (50 g) under cooling with ice. The reaction mixture was stirred for 1 hour. The reaction mixture was diluted with tetrahydrofuran (350 mL), mixed with anhydrous magnesium sulfate (90 g) and stirred at room temperature for 30 minutes. The thus obtained insoluble was filtered and washed with tetrahydrofuran (350 mL). All of the filtrates were cooled with ice and pyridine (42 mL) was added thereto. Pivaloylchloride (50 g) was gradually added dropwise to the reaction mixture. The reaction mixture was stirred for 15 minutes under cooling with ice. To the reaction mixture, 1 N hydrochloric acid (200 mL) was added, followed by extraction twice with ethyl acetate. The extract was washed with water, a saturated aqueous sodium bicarbonate solution, water and a saturated aqueous sodium chloride solution, dried over anhydrous magnesium sulfate and then concentrated to obtain the title compound (76.8 g) having the following physical properties.
TLC: Rf 0.53 and 0.57 (chloroform : methanol = 10:1)
NMR (CDCl₃): δ 8.68 (d, J = 5.5 Hz) and 8.55 (d, J = 5.5 Hz) (total 1H), 8.40 (d, J = 5.5 Hz) and 8.23 (d, J = 5.5 H) (total 1H), 3.60 (d, J = 4.8 Hz) and 3.36 (d, J = 2.2 Hz) (total 1H), 2.97 (dd, J = 2.2 and 6.6 Hz) and 2.90 (dd, J = 4.8 and 9.5 Hz) (total 1H), 1.75-1.55 (m, 1H), 1.27 and 1.26 (each s, total 9H), 1.14 and 1.03 and 1.00 and 0.97 (each d, both J = 6.6 Hz, total 6H)

### Reference Example 2

### N-(3-Azido-2-hydroxy-4-methyl)pentanoyl-N'-(2,2-dimethylpropionyl)hydrazine

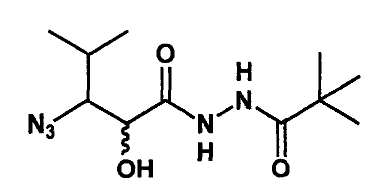

A mixture of the compound prepared in Reference Example 1 (76.8 g), sodium azide (32.8 g) and magnesium chloride hexahydrate (51.3 g) was refluxed for 14.5 hours in methanol (670 mL). The reaction mixture was cooled with ice, and ethyl acetate (1 L) and 1 N hydrochloric acid (1 L) were added thereto, followed by separation to layers. The water layer was extracted twice with ethyl acetate and mixed with the organic layers. The reaction mixture was washed with water, a saturated aqueous sodium bicarbonate solution, water and a saturated aqueous sodium chloride solution, dried over anhydrous magnesium sulfate and then concentrated to obtain the title compound (88.2 g) having the following physical properties.
TLC: Rf 0.40 and 0.44 (chloroform : methanol = 10:1)
NMR (CDCl₃): δ 9.31 (d, J = 5.1 Hz) and 9.13 (d, J = 5.1 Hz) (total 1H), 8.36 (d, J = 5.1 Hz) and 8.23 (d, J = 5.1 Hz) (total 1H), 4.5-4.35 (m, 2H), 3.47-3.38 (m, 1H), 2.2-2.0 (m, 1H), 1.28 (s) and 1.27 (s) (total 9H), 1.11 (d, J = 6.6 Hz) and 1.06 (d, J = 7.0 Hz) and 1.03 (d, J = 6.6 Hz) and 1.02 (d, J = 7.0 Hz) (total 6H)

### Example 1

### 1-(5-(t-Butyl)-1,3,4-oxadiazol-2-yl)-3-methyl-2-azidobutanol:

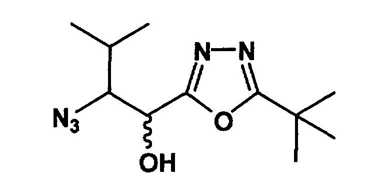

Pyridine (92 mL) was added to and dissolved in to t-butyl methyl ether (300 mL) suspension of the compound prepared in Reference Example 2 (88.2 g). Trimethylsilyl chloride (45 mL) was added dropwise to the solution at room temperature. The reaction mixture was stirred at room temperature for 30 minutes. The reaction mixture was cooled with ice, and thionyl chloride (26 mL) was gradually added dropwise thereto, followed by stirring for 30 minutes under cooling with ice. The reaction mixture was mixed with anhydrous magnesium sulfate (88 g), followed by stirring at room temperature for 30 minutes. The reaction mixture was filtered and washed with t-butyl methyl ether (500 mL), and the filtrate was concentrated. The residue was dissolved in toluene (325 mL) and refluxed for 20 minutes. The mixture was cooled to room temperature, and methanol (200 mL) and potassium fluoride (20.8 g) were added thereto, followed by stirring for 30 minutes. The reaction mixture was concentrated, and the residue was dissolved in t-butyl methyl ether (300 mL), washed with water, 1 N hydrochloric acid (twice), water, a saturated aqueous sodium bicarbonate solution, water and a saturated aqueous sodium chloride solution in this order, dried over anhydrous magnesium sulfate and then concentrated. The residue was dissolved in methanol (200 mL), the solution was mixed with activated carbon (10 g), allowed to stand for 30 minutes and filtered, and then activated carbon was washed with methanol and the filtrate was concentrated to obtain the compound of the present invention (74.2 g) having the following physical properties.
TLC: Rf 0.24 (hexane : ethyl acetate = 2:1)
NMR (CDCl₃): δ 5.07 (dd, J = 4.4, 7.3 Hz) and 4.94 (t, J = 7.3 Hz) (total 1H), 3.93 (br d, J = 7.3 Hz) and 3.36 (br d, J = 7.3 Hz) (total 1H), 3.68 (dd, J = 5.1, 7.3 Hz) and 3.52 (dd, J = 4.4, 7.0 Hz) (total 1H), 2.16 and 1.96 (each m, total 1H), 1.44 (s, 9H), 1.10 (d, J = 7.0 Hz) and 1.08 (d, J = 6.6 Hz) and 1.05 (d, J = 7.0 Hz) and 0.99 (d, J = 6.6 Hz) (total 6H)

### Example 2

### 1-(5-(t-Butyl)-1,3,4-oxadiazol-2-yl)-3-methyl-2-aminobutanol:

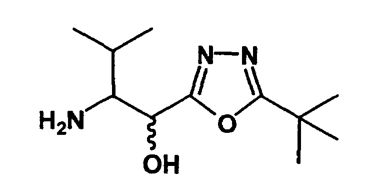

A 50% hydrous product of 10% palladium-carbon (Pd-C) (16 g) was added to a methanol (300 mL) solution of the compound prepared in Example 1 (74.2 g). The reaction mixture was stirred for 5.5 hours under a hydrogen. The reaction mixture was filtered and the filtrate was concentrated. The residue was dissolved in methanol (200 mL), activated carbon (10 g) was added thereto, the mixture was filtered, and the filtrate was concentrated. Hexane was added to the residue, and the solid was collected by filtration, washed with hexane and then dried to obtain the compound of the present invention (48.8 g) having the following physical properties.
TLC: Rf 0.22 (chloroform: methanol = 10:1)
NMR (CDCl₃): δ 4.92 (d, J = 5.5 Hz) and 4.74 (d, J = 4.0 Hz) (total 1H), 3.10 (dd, J= 4.3, 6.2 Hz) and 2.71 (dd, J = 5.5, 8.8 Hz) (total 1H), 1.85-1.45 (m, 1H), 1.43 (s) and 1.42 (s) (total 9H), 1.00 (d, J = 6.6 Hz) and 0.98 (d, J = 6.6 Hz) and 0.94 (d, J = 6.6 Hz) (total 6H)

### INDUSTRIAL APPLICABILITY

According to the present invention, preparation of intermediates (compounds of formula (III)) of 1,3,4-oxadiazole derivatives useful as pharmaceuticals (elastase inhibitors) can be prepared efficiently via novel synthesis intermediates (compounds of formula (I)), without requiring low temperature reactions such as the conventional processes and by smaller number of steps.

## Claims

1. A process for a preparation of a 1,3,4-oxadiazole derivative represented by formula (I): 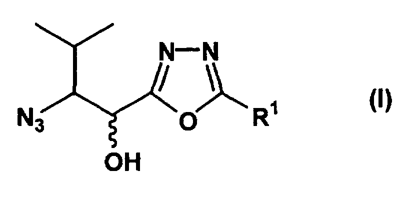 wherein R¹ has the same meaning as described below, which comprises:
subjecting a compound represented by formula (II): 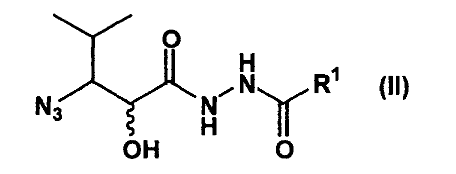 wherein R¹ represents a phenyl group or 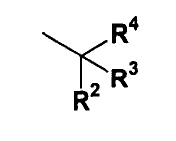 wherein R², R³ and R⁴ each independently represents (1) a hydrogen atom, (2) a C1-8 alkyl group, (3) a C3-7 cycloalkyl group, (4) a phenyl group, (5) a phenyl group substituted with 1 to 3 substituents selected from a C1-8 alkyl group, a C1-8 alkoxy group, a halogen atom, a trifluoromethyl group and a trifluoromethoxy group or (6) a 3,4-methylenedioxyphenyl group, or (7) R² and R³ are taken together to represent a C2-6 alkylene group,
to an introducing reaction of a protecting group of a hydroxyl group, a cyclization reaction and a deprotection reaction of a protecting group of a hydroxyl group.

2. A process for a preparation of a 1,3,4-oxadiazole derivative represented by formula (III) or a salt thereof: 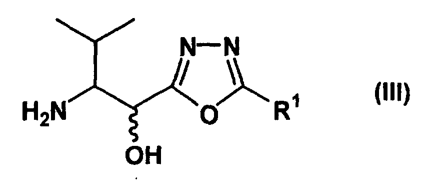 wherein R¹ has the same meaning as described below, which comprises:
subjecting a compound represented by formula (II): 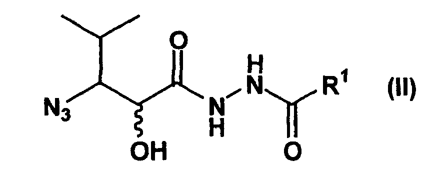 wherein R¹ has the same meaning as described in claim 1,
to an introducing reaction of a protecting group of a hydroxyl group, a cyclization reaction and a deprotection reaction of a protecting group of a hydroxyl group to obtain a 1,3,4-oxadiazole derivative represented by formula (I): 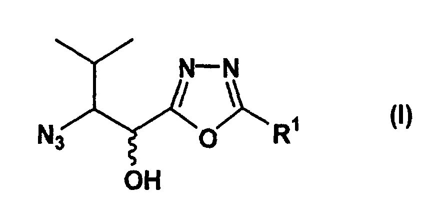 wherein R¹ has the same meaning as described above, and
subjecting the 1,3,4-oxadiazole derivative represented by formula (I) to a reduction reaction, optionally followed by conversion to a salt.

3. A process for a preparation of a 1,3,4-oxadiazole derivative represented by formula (III) or a salt thereof: 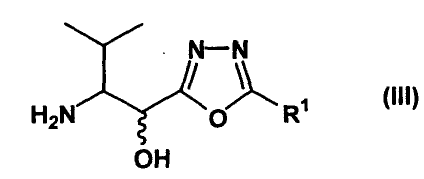 wherein R¹ has the same meaning as described below, which comprises:
subjecting a 1,3,4-oxadiazole derivative represented by formula (I): 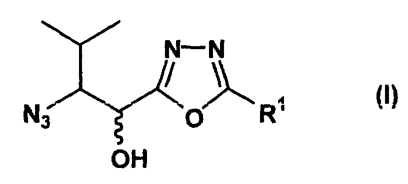 wherein R¹ has the same meaning as described in claim 1,
to a reduction reaction, optionally followed by conversion to a salt.

4. A 1,3,4-oxadiazole derivative represented by formula (I): 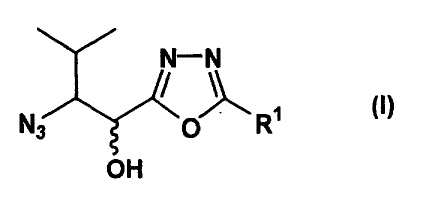 wherein R¹ has the same meaning as described in claim 1.

5. The 1,3,4-oxadiazole derivative according to claim 4, which is 1-(5-(t-butyl)-1,3,4-oxadiazol-2-yl)-3-methyl-2-azidobutanol.
